# EUROPEAN PATENT APPLICATION

(11) **EP 1 518 508 A1**
(43) Date of publication of application: **30.03.2005**
(21) Application number: 04254525.1
(22) Date of filing: 28.07.2004
(51) Int. Cl.: A61B 19/00

(54) **Tracking array to create multiple spatial tracking configurations for a surgical navigation system**

(30) Priority: 27.08.2003 US 649749
(71) Applicant: Zimmer Orthopaedic Surgical Products, Dover, Ohio 44622 (US)
(72) Inventor: Harmon, Kim R., NE, Mineral City Ohio 44656 (US); Donaldson, Timothy A., Massillon Ohio 44646 (US); Barker, Richard L., Scio Ohio 43988 (US)
(74) Representative: Mays, Julie

(57) **Abstract**

A tracking array (10) for attachment to an object in a surgical environment includes tracking elements (48) detectable by a surgical navigation system to permit tracking of the object.

## Description

### BACKGROUND

The present invention relates to devices used with surgical navigation systems. In particular, the present invention relates to an improved array of tracking elements attachable to an object to permit the surgical navigation system to track the position and orientation of the object during a surgical procedure.

Many surgical procedures are now performed with surgical navigation systems in which sensors detect tracking elements attached in known relationship to an object in the surgical suite such as a surgical instrument, implant, or patient body part. The sensor information is fed to a computer that then triangulates the position of the tracking elements within the surgical navigation system coordinate system. Thus the computer can resolve the position and orientation of the object and display the position and orientation for surgeon guidance. For example, the position and orientation can be shown superimposed on an image of the patient's anatomy obtained via X-ray, CT scan, ultrasound, or other imaging technology.

It is desirable for the surgical navigation system to be able to recognize a particular tracking array and associate it with a particular object being tracked. The ability to distinguish individual tracking arrays is especially important in surgical procedures involving the tracking of multiple objects simultaneously.

### SUMMARY

The present invention provides a tracking array for attachment to an object in a surgical environment. The tracking array includes tracking elements detectable by a surgical navigation system to permit tracking of the object.

In one aspect of the invention, a surgical navigation system includes means for tracking an object by detecting the positions of a predetermined number of tracking elements. An array body attached to the object has a predetermined number of tracking element attachment locations greater than the predetermined number of tracking elements. The predetermined number of tracking elements is attached to the array body in alternate predetermined spatial configurations by attaching the tracking elements to different subsets of the tracking element attachment locations. Each of the alternate predetermined spatial configurations is uniquely identifiable by the means for tracking such that each of the alternate predetermined spatial configurations can be used to identify different objects to which the tracking elements are attached.

In another aspect of the invention, a set of tracking arrays for a surgical navigation system includes first and second array bodies having a predetermined number of tracking element attachment locations greater than the predetermined number of tracking elements in each array. The tracking element attachment locations are arranged in a predetermined spatial pattern. The first array body has a first group of tracking elements attached to the tracking element attachment locations to create an array of tracking elements in a predetermined spatial configuration. The second array body has a second group of tracking elements attached to the tracking element attachment locations to create a second array of tracking elements in a predetermined spatial configuration distinct from the predetermined spatial configuration of the first array of tracking elements.

In another aspect of the invention, a multiple configuration tracking array for use with a surgical navigation system includes a predetermined number of tracking elements recognizable by the surgical navigation system to provide position information. An array body has a number of tracking element attachment locations greater than the predetermined number of tracking elements. The predetermined number of tracking elements is positionable in alternate configurations of attachment locations to produce alternate patterns distinguishable by the surgical navigation system such that different configurations may be correlated to different tracked objects within the system.

In another aspect of the invention a surgical navigation system for tracking an object during a surgical procedure includes means for tracking an object by detecting the positions of a predetermined number of tracking elements. Each tracking element has a planar reflective surface. An array body is attached to the object and has a predetermined number of tracking element attachment locations. The predetermined number of tracking elements is attachable to the array body in a predetermined spatial configuration identifiable by the means for tracking such that the predetermined spatial configuration can be used to identify different objects to which the tracking elements are attached.

In another aspect of the invention a method of making a tracking array for use with a surgical navigation system includes providing a first set of a predetermined number of tracking elements; providing a first array body having a predetermined number of tracking element attachment locations greater than the predetermined number of tracking elements, the tracking element attachment locations being arranged in a spatial pattern; and attaching the first predetermined number of tracking elements to a subset of the predetermined number of tracking element attachment locations of the first array body to form a first spatial arrangement of tracking elements attached to the first array body.

### BRIEF DESCRIPTION OF THE DRAWINGS

Various embodiments of the present invention will be discussed with reference to the appended drawings. These drawings depict only illustrative embodiments of the invention and are not to be considered limiting of its scope.
FIG. 1 is a top plan view of an illustrative array body according to the present invention.
FIG. 2 is a top plan view of the array body of FIG. 1 diagrammatically showing a unique configuration of tracking elements attached to the body.
FIG. 3 is a top plan view of the array body of FIG. 1 diagrammatically showing a unique configuration of tracking elements attached to the body.
FIG. 4 is a perspective view of an array made with the array body of FIG. 1 and an alternative type of tracking element.
FIG. 5 is a perspective view of an array made with the array body of FIG. 1 and an alternative type of tracking element.
FIG. 6 is a side elevation view of the array of FIG. 5 mounted on a surgical instrument.

### DESCRIPTION OF THE ILLUSTRATED EMBODIMENTS

Embodiments of a tracking array for use with a surgical navigation system include an array body and tracking elements attached to the array body. The tracking elements are detectable by the surgical navigation system such that the three dimensional position of the tracking elements can be related to a surgical navigation coordinate system. For example, the surgical navigation system may include multiple sensors at known locations that feed tracking element position information to a computer. The computer may then use the position information from the multiple sensors to triangulate the position of each tracking element within the surgical navigation coordinate system. The tracking array may be attached to an object such as a surgical instrument, implant, or patient body part in a known orientation. The surgical navigation system can then determine the position and orientation of the object by detecting the position and orientation of the tracking array and then resolving the position and orientation of the object from the known relationship between the tracking array and the object.

The tracking elements may be detectable by imaging, acoustically, electromagnetically, or by other suitable detection means. Furthermore, the tracking elements may be active or passive. Examples of active tracking elements may include light emitting diodes in an imaging system, ultrasonic emitters in an acoustic system, and electromagnetic field emitters in an electromagnetic system. Examples of passive tracking elements may include elements with reflective surfaces.

A single tracking element may identify a point within the surgical navigation coordinate system. A pair of tracking elements may identify a line within the surgical navigation coordinate system. Three tracking elements may identify a plane within the surgical navigation coordinate system. With three tracking elements, the position and orientation of an object connected to the tracking array may be determined. By providing more than three tracking elements, redundant identification of a plane within the surgical navigation coordinate system is possible so that if one of the redundant tracking elements should become momentarily blocked from detection, the surgical navigation system may continue to track the tracking array and associated object. The surgical navigation system is configured to track a predetermined number of tracking elements for each array, including redundant tracking elements if present.

The tracking array may be provided with the tracking elements arranged in a predetermined spatial arrangement that not only permits tracking of the array but also identification of a particular array by detecting the predetermined spatial arrangement. The tracking array may be a multiple configuration array in which a common array body may accommodate the positioning of the tracking elements in different unique spatial arrangements. Thus, the one array body geometry can be produced inexpensively in large quantities and used to assemble individually unique array configurations. The surgical navigation system may identify different objects in the surgical navigation coordinate system by detecting the unique tracking element spatial configuration of a particular array. The multiple configuration array may include an array body having a number of tracking element attachment locations greater than the predetermined number of tracking elements associated with each array. The tracking elements may be attached to the array body in alternate spatial configurations by attaching the tracking elements to different subsets of the tracking element attachment locations to create uniquely identifiable tracking arrays from the same array body and tracking elements. The number of tracking elements may exceed the minimum number required to identify the position and orientation of the tracked object so that redundant information is provided. The spatial arrangement of the tracking elements may be such that if one of the redundant tracking elements is blocked from detection, the remaining tracking elements still form a unique spatial arrangement.

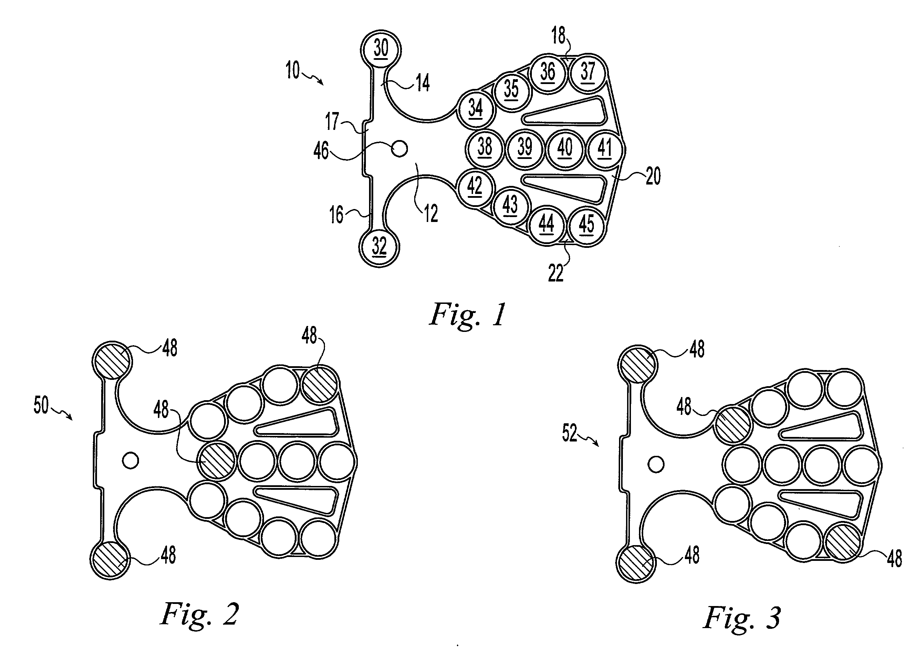

FIG. 1 depicts an illustrative array body 10 having a palmate layout including a central portion 12 and first 14, second 16, third 18, fourth 20, and fifth 22 extensions, or fingers, extending outwardly from the central portion 12. The first 14 and second 16 extensions are collinearly aligned to form a base 17 of the array body 10. Each of the first 14 and second 16 extensions includes a tracking element attachment location 30, 32. The third extension 18 includes four tracking element attachment locations 34, 35, 36, and 37. The fourth extension 20 also includes four tracking element attachment locations 38, 39, 40, and 41. The fifth extension 22 also includes four tracking element attachment locations 42, 43, 44, and 45. Thus, the array body includes fourteen tracking element attachment locations. An attachment hole 46 facilitates attaching the array body 10 to an object to be tracked.

Tracking elements 48 may be attached to the tracking element attachment locations to form tracking arrays 50, 52 as shown in FIGS. 2 and 3. FIG. 2 depicts a uniquely configured tracking array 50 in which tracking elements are attached to a subset of four 30, 32, 37, 38 of the fourteen tracking element attachment locations to form a predetermined spatial configuration. FIG. 3 depicts an alternate uniquely configured tracking array 52 in which tracking elements 48 are attached to a different subset 30, 32, 34, 35 of four of the tracking element attachment locations to form an alternative unique predetermined spatial array. The alternately configured arrays 50, 52 of FIGS. 2 and 3 may be attached to different objects to be tracked by the surgical navigation system. The alternate configuration permits the surgical navigation system to identify individual arrays 50, 52 and thus the different objects to which they are attached. By providing a common array body 10 as the base for a variety of array configurations 50, 52, the arrays 50, 52 may be quickly and inexpensively manufactured. A single manufacturing setup produces a quantity of array bodies 10 which are configured as needed. This saves time and money in manufacturing by reducing the amount of tooling required and eliminating the need to change equipment setups to produce bodies 10 with different geometries. The body 10 may be machined, molded, or otherwise formed. The body 10 may be made of metal, polymers, or other suitable materials. For example, the body 10 may be injection molded from a polymer resin to quickly and inexpensively form a quantity of bodies 10. The arrays 50, 52 may then be configured by attaching tracking elements 48 to the bodies 10. The tracking elements 48 may be attached during manufacturing or by an end user. The tracking elements 48 may be releasably attached to permit a particular array to be reconfigured or the tracking elements may be permanently attached. Attachment methods may include snap-fitting, press-fitting, gluing, welding, threading, or other suitable releasable or permanent attachment.

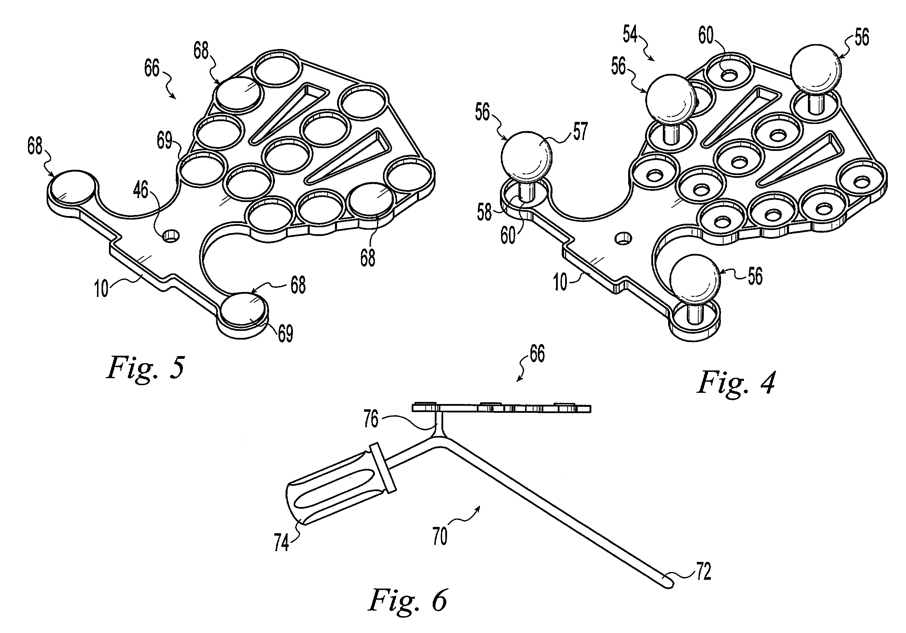

The illustrative arrays 54, 66 of FIGS. 4 and 5 include optically tracked passive tracking elements 56, 68; however, other types of tracking elements are contemplated and fall within the scope of the present invention. The tracking elements 56 of FIG. 4 have a spherical reflective surface 57 for reflecting predetermined wavelengths of light detectable by the surgical navigation system. For example, the reflective surface 57 may reflect infrared wavelengths to reduce the chance of a false detection from reflected visible light in the surgical navigation environment. The tracking elements 56 are attached to the array body 10 by posts 58 extending from the tracking elements 56 and engaging holes 60 formed in the body 10 at the tracking element attachment location. The tracking elements 68 of FIG. 5 have a circular planar reflective surface 67. The tracking elements 68 have cylindrical bodies that engage cylindrical depressions 69 in the array body 10. The circular planar reflective surface 67 may be manufactured less expensively than the spherical surface 57 of FIG. 4. For example, a circular planar reflective surface 67 may be punched out of a sheet of reflective material with a die cutter. The spherical surface 57 requires cutting two circular pieces of reflective material and forming them into hemispherical surfaces. The hemispherical surfaces must then be joined together to form the spherical surface.

The cost saving features of the illustrative arrays 50, 52, 54, 66, allows them to be made disposable. Disposability is beneficial since each procedure is conducted with a new array. Thus, no damage from handling or cleaning in prior surgical procedures will impair the accuracy of tracking or identifying the arrays in subsequent surgical procedures. Making the arrays disposable further lowers the cost of the arrays by permitting the use of less expensive materials that are not able to withstand cleaning and sterilization.

FIG. 6 depicts the tracking array 66 of FIG. 5 attached to a surgical instrument 70. The surgical instrument 70 includes a working end 72 and a handle 74 for manipulating the working end. An attachment post 76 extends from the surgical instrument 70 to engage the attachment hole 46 such that the tracking array 66 and surgical instrument 70 are in fixed known relationship. By tracking the array 66, the surgical navigation system can resolve the position of the surgical instrument 70 to aid the surgeon in performing a surgical procedure.

The illustrated arrays 50, 52, 54, 66 also demonstrate an illustrative identification scheme. The illustrative identification scheme utilizes four tracking elements to provide a redundant number of tracking elements to identify position and orientation. As long as three tracking elements are detected by the surgical navigation system, the array may be tracked. Furthermore, the identification scheme follows a set of rules such that if any three of the four tracking elements in each array are visible, the array presents a unique spatial pattern for identification. The first rule requires that tracking elements 48, 68, 56 be attached to the tracking element attachment locations 30, 32 on the first 14 and second 16 extensions in each configuration. These two tracking elements identify the base 17 of the array in each configuration. Tracking elements 48, 68, 56 are also attached at two additional locations selected from the remaining attachment locations 34-45. The second rule requires that the remaining locations 34-45 are arranged so that none of them form an equilateral triangle with the base locations 30, 32. In other words, there is no tracking element attachment location equidistant from the base attachment locations 30, 32. The third rule requires that no two of the remaining locations 34-45 are collinear with either of the base tracking element attachment locations 30, 32. In other words, a line cannot be drawn from the center of either of the base tracking element attachment locations 30, 32 through the centers of two other tracking element attachment locations 34-45. The fourth rule requires that two tracking element attachment locations on the same extension 18, 20, 22 are never used in the same configuration. In other words, the four tracking element attachment locations 34-37 on the third extension 18 are mutually exclusive as to any particular array. This is true for the tracking element attachment locations 38-41, 42-45 on the fourth 20 and fifth 22 extensions also. The fifth rule requires that none of the remaining locations 34-45 is used in more than one array configuration. In other words, once one of the remaining tracking element attachment locations 34-45 has been used in one array configuration, it cannot be used in a different array configuration. This prevents an array configuration from being misidentified if one of the redundant tracking elements is blocked from detection by the surgical navigation system. In the illustrative identification scheme, six different configurations of four tracking elements may be formed using the illustrative array body 10 and following these rules. For each configuration, if any one of the four tracking elements is blocked from detection, the remaining three tracking elements will present a uniquely identifiable configuration. Thus, each array configuration of four tracking elements contains four unique sub configurations of three tracking elements each for a total of 30 unique spatial patterns. The identification associated with each array configuration thus includes a total of five unique spatial patterns; the unique four element configuration and each of the unique three element sub configurations. If any one of the five spatial patterns associated with a particular configuration is identified by the surgical navigation system, then the particular array, and consequently the object to which it is attached, is positively identified. Other schemes can be designed to provide unique patterns for identification within the scope of this invention. Schemes with no redundancies or multiple redundancies are also contemplated and fall within the disclosed invention.

Although embodiments of a tracking array and its use have been described and illustrated in detail, it is to be understood that the same is intended by way of illustration and example only and is not to be taken by way of limitation. Accordingly, variations in and modifications to the tracking array and its use will be apparent to those of ordinary skill in the art, and the following claims are intended to cover all such modifications and equivalents.

## Claims

1. A surgical navigation system comprising:
means for tracking an object by detecting the positions of a predetermined number of tracking elements; and
an array body attached to the object, the array body having a predetermined number of tracking element attachment locations greater than the predetermined number of tracking elements, each of the predetermined number of tracking elements being attachable to one of the tracking element attachment locations, the predetermined number of tracking elements being attachable to the array body in alternate predetermined spatial configurations by attaching the tracking elements to different subsets of the tracking element attachment locations, each of the alternate predetermined spatial configurations being uniquely identifiable by the means for tracking such that each of the alternate predetermined spatial configurations can be used to identify different objects to which the tracking elements are attached.

2. The surgical navigation system of claim 1 wherein the tracking elements have planar reflective surfaces detectable by the means for tracking.

3. The surgical navigation system of claim 1 wherein the predetermined number of tracking elements is greater than the minimum number required by the means for tracking to track the object such that redundant tracking information is provided, each of the alternate predetermined spatial configurations further containing unique sub configurations uniquely identifiable if one of the redundant tracking elements is blocked from detection by the means for tracking.

4. A set of tracking arrays for a surgical navigation system that tracks individual arrays by detecting a predetermined number of tracking elements in each array, the set of tracking arrays comprising:
a first array body having a predetermined number of tracking element attachment locations greater than the predetermined number of tracking elements in each array, the tracking element attachment locations being arranged in a predetermined spatial pattern;
a first group of tracking elements equaling in number the predetermined number of tracking elements, each of the tracking elements in the first group of tracking elements being attached to one of the tracking element attachment locations of the first array body to create an array of tracking elements in a predetermined spatial configuration, a second array body having a predetermined number of tracking element attachment locations greater than the predetermined number of tracking elements in each array, the tracking element attachment locations being arranged in the same predetermined spatial pattern as those of the first array body;
a second group of tracking elements equaling in number the predetermined number of tracking elements, each of the tracking elements in the second group of tracking elements being attached to one of the tracking element attachment locations of the second array body to create a second array of tracking elements in a predetermined spatial configuration distinct from the predetermined spatial configuration of the first array of tracking elements.

5. The set of tracking arrays of claim 4 wherein each of the first and second arrays of tracking elements is arranged according to a redundant identification scheme such that if any one of the tracking elements in the array is blocked from detection, the remaining tracking elements in the array still form a uniquely identifiable pattern to positively identify the array within the surgical navigation system.

6. A multiple configuration tracking array for use with a surgical navigation system, the surgical navigation system being able to track the array to determine the position of objects to which the array is attached, the array comprising:
a predetermined number of tracking elements recognizable by the surgical navigation system to provide position information; and
an array body having a number of tracking element attachment locations greater than the predetermined number of tracking elements, the predetermined number of tracking elements being positionable in alternate configurations of attachment locations to produce alternate patterns distinguishable by the surgical navigation system such that different configurations may be correlated to different tracked objects within the system.

7. The multiple configuration tracking array of claim 6 wherein the array body is molded from a polymer.

8. The multiple configuration tracking array of claim 6 wherein the array body includes a cylindrical recess at each of the tracking element attachment locations and the tracking elements include cylindrical bodies engageable with the cylindrical recesses.

9. The multiple configuration tracking array of claim 6 wherein the tracking elements emit light that is tracked by the surgical navigation system.

10. The multiple configuration tracking array of claim 6 wherein the tracking elements include reflective surfaces that are tracked by the surgical navigation system.

11. The multiple configuration tracking array of claim 6 wherein the tracking elements emit sonic waves that are tracked by the surgical navigation system.

12. The multiple configuration tracking array of claim 6 wherein the tracking elements emit electromagnetic field energy that is tracked by the surgical navigation system.

13. A surgical navigation system for tracking an object during a surgical procedure, the system comprising:
means for tracking an object by detecting the positions of a predetermined number of tracking elements, each tracking element having a planar reflective surface; and
an array body attached to the object, the array body having a predetermined number of tracking element attachment locations, each of the predetermined number of tracking elements being attachable to one of the tracking element attachment locations, the predetermined number of tracking elements being attachable to the array body in a predetermined spatial configuration identifiable by the means for tracking such that the predetermined spatial configuration can be used to identify different objects to which the tracking elements are attached.

14. A method of making a tracking array for use with a surgical navigation system, the method comprising:
providing a first set of a predetermined number of tracking elements;
providing a first array body having a predetermined number of tracking element attachment locations greater than the predetermined number of tracking elements, the tracking element attachment locations being arranged in a spatial pattern; and
attaching the first predetermined number of tracking elements to a subset of the predetermined number of tracking element attachment locations of the first array body to form a first spatial arrangement of tracking elements attached to the first array body.

15. The method of claim 14 further comprising:
providing a second set of tracking elements having the same predetermined number as the first set;
providing a second array body having the same number and pattern of tracking element attachment locations as the first array body; and
attaching the second set of tracking elements to a subset of the tracking element attachment locations of the second array body to form a second spatial arrangement of tracking elements attached to the second array body, the second spatial arrangement of tracking elements being distinct from the first spatial arrangement of tracking elements.
